# EUROPÄISCHE PATENTANMELDUNG

(11) **EP 1 186 587 A1**
(43) Veröffentlichungstag der Anmeldung: **13.03.2002**
(21) Anmeldenummer: 01120695.0
(22) Anmeldetag: 04.09.2001
(51) Int. Cl.: C07C 39/11, C07C 37/055, C07C 37/00

(54) **Verfahren zur Herstellung von 3-Hydroxybenzylalkohol**

(30) Priorität: 12.09.2000 DE 10044910
(71) Anmelder: BAYER AG, 51368 Leverkusen (DE)
(72) Erfinder: Mais, Franz-Josef, Dr., 40591 Düsseldorf (DE); Diehl, Herbert, Dr., 51373 Leverkusen (DE)

(57) **Zusammenfassung**

3-Hydroxybenzylalkohol kann auf einfache und kostengünstige Weise hergestellt werden, wenn man (3-Chlormethylphenyl)-chlorformiat mit einer Carbonsäure und/oder einem Carbonsäuresalz einer Acidolyse unterwirft und anschließend mit dem Reaktionsprodukt dieser ersten Stufe eine Alkoholyse durchführt.

## Beschreibung

Die vorliegende Erfindung betrifft ein Verfahren zur Herstellung von 3-Hydroxybenzylalkohol aus (3-Chlormethylphenyl)-chlorformiat.

3-Hydroxybenzylalkohol ist ein wertvolles Zwischenprodukt zur Herstellung von pharmazeutischen Wirkstoffen.

Es sind verschiedene Verfahren zur Herstellung von 3-Hydroxybenzylalkohol bekannt. Diese gehen von 3-Hydroxybenzaldehyd, 3-Hydroxybenzoesäure oder Derivaten dieser Verbindungen aus. Zur Überführung in 3-Hydroxybenzylalkohol werden diese Ausgangsprodukte reduziert.

Z.B. kann man 3-Hydroxybenzoesäure mit Lithiumaluminiumhydrid, mit organischen Aluminium- oder Bor-Verbindungen oder mittels Elektrolyse zum 3-Hydroxybenzylalkohol reduzieren.

Ähnlich ist die Reduktion von 3-Hydroxybenzoesäureestem zu 3-Hydroxybenzylalkohol, die gegebenenfalls unter Verwendung von Aluminium- oder Borhydriden durchgeführt werden kann.

Für die Reduktion von 3-Hydroxybenzaldehyd zum 3-Hydroxybenzylalkohol kann man folgende Reagenzien einsetzen: Lithiumaluminiumhydrid, Natriumborhydrid, Zinkborhydrid, Zinndichlorid plus Magnesium, Natriumamalgam oder polymer gebundenes Nicotinamid-adenin-dinucleotid in der H-Form (NADH). Man kann diese Reaktion auch als katalytische Hydrierung durchführen, z.B. mit Raney-Nickel-Katalysatoren oder Platin-Katalysatoren.

Ähnlich ist die katalytische Reduktion von Benzylethern des 3-Hydroxybenzylalkohol.

Weitere Bildungsweisen von 3-Hydroxybenzylalkohol sind Spaltungen von (3-Hydroxyphenyl)-methylformiat und (3-Hydroxyphenyl)-methyl-2-nitrobenzolsulfenat.

Nachteilig bei allen diesen Verfahren ist, dass als Ausgangsmaterial nur relativ teure Stoffe wie 3-Hydroxybenzoesäure, 3-Hydroxybenzaldehyd oder Derivate davon eingesetzt werden können. Außerdem müssen häufig Reduktionsmittel oder katalytische Hydrierungen angewendet werden, die besonderen Aufwand erfordern oder teuer sind.

Es besteht daher nach wie vor ein Bedürfnis nach einem Verfahren zur einfachen und kostengünstigen Herstellung von 3-Hydroxybenzylalkohol.

Es wurde nun ein Verfahren zur Herstellung von 3-Hydroxybenzylalkohol gefunden, das dadurch gekennzeichnet ist, dass man (3-Chlormethylphenyl)-chlorformiat mit einer Carbonsäure und/oder einem Carbonsäuresalz einer Acidolyse unterwirft und anschließend mit dem Reaktionsprodukt dieser ersten Stufe eine Alkoholyse durchführt.

Das erfindungsgemäß einzusetzende (3-Chlormethylphenyl)-chlorformiat ist literaturbekannt. Es kann beispielsweise durch Phosgenierung von m-Kresol und anschließender Seitenkettenchlorierung des so erhaltenen (3-Methylphenyl)-chlorformiats erhalten werden. Als Katalysatoren für die Phosgenierung können z.B. Dimethylformamid und Phosphorverbindungen wie Triphenylphosphin verwendet werden. Als Zusätze bei der Seitenkettenchlorierung des (3-Methylphenyl)-chlorformiats kann man bekannte Zusätze, z.B. Pyridin, Pyridinderivate wie Methyl- oder Chlorpyridine, Amide wie Acetamid oder Benzamid, Biuret, Harnstoffe, cyclische Lactame, Urethane, Amine oder Polyamine wie Urotropin, Ethanolamin oder Tetraethylenpentamin, Phosphorverbindungen wie Phosphortrichlorid oder Phosphorpentachlorid oder Schwefelverbindungen wie Diarylsulfide und Diaryldisulfide einsetzen.

Als Chlorierungsmittel bei der Seitenkettenchlorierung des (3-Methylphenyl)-chlorformiats kann man beispielsweise Chlor oder Sulfurylchlorid einsezten.

In das erfindungsgemäße Verfahren wird vorteilhafterweise (3-Chlormethylphenyl)-chlorformiat eingesetzt, das weniger als 1 Gew.-% nicht seitenkettenchloriertes (3-Methylphenyl)-chlorformiat und weniger als 5 Gew.-% höherchloriertes (3-Bis-chlormethylphenyl)-chlorformiat enthält.

Das (3-Chlormethylphenyl)-chlorformiat wird erfindungsgemäß mit einer Carbonsäure und/oder einem Carbonsäuresalz acidolysiert. Als Produkt dieser Umsetzung erhält man den Bisester des 3-Hydroxybenzylalkohols mit der eingesetzten Carbonsäure.

Als Carbonsäure kann man erfindungsgemäß eine breite Pallette von aliphatischen, cycloaliphatischen und aromatischen Carbonsäuren einsetzen. Als aliphatische Carbonsäuren kommen z.B. Carbonsäuren mit linearen oder verzweigten Alkylresten mit 1 bis 12 C-Atomen, bevorzugt 1 bis 6 C-Atomen, in Frage. Als Beispiele seien genannt: Ameisensäure, Essigsäure, Propionsäure, Buttersäure und 2-Methylpropionsäure.

Als cycloaliphatische Carbonsäuren kommen z.B. solche mit 4 bis 11 C-Atomen, bevorzugt 4 bis 7 C-Atomen, in Frage. Als Beispiele seien genannt: Cyclopropancarbonsäure, Cyclopentancarbonsäure und Cyclohexancarbonsäure.

Als aromatische Carbonsäuren können z.B. solche mit 7 bis 12 C-Atomen, bevorzugt 7 bis 10 C-Atomen eingesetzt werden. Als Beispiel sei Benzoesäure genannt.

Die aliphatischen und cycloaliphatischen Carbonsäuren können gegebenenfalls noch z.B. ein- bis dreifach substituiert sein, beispielsweise mit Halogenatomen wie Fluor, Chlor und/oder Brom, bevorzugt mit Fluor und/oder Chlor.

Die aromatischen Carbonsäuren können ebenfalls z.B. ein- bis dreifach substituiert sein, beispielsweise mit Halogenatomen wie Fluor, Chlor und/oder Brom und/oder C₁-C₆-Alkylgruppen wie Methyl, Ethyl, n-Propyl, i-Propyl, n-Butyl, t-Butyl, Hexyl und/oder Cyclohexyl.

Als Carbonsäuresalze können z.B. Salze von Alkalimetallen und Erdalkalimetallen und Ammoniumsalze eingesetzt werden. Bevorzugt sind Alkalimetallsalze wie Natriumsalze und Ammoniumsalze. Der Säureteil der Carbonsäuresalze kann den zuvor beschriebenen aliphatischen, cycloaliphatischen und aromatischen Carbonsäuren entsprechen.

Die Menge der einzusetzenden Carbonsäure und/oder des einzusetzenden Carbonsäuresalzes hängt von der Art und Weise ab, wie die Reaktionspartner (3-Chlormethylphenyl)-chlorformiat und Carbonsäure und/oder Carbonsäuresalz in Kontakt gebracht werden.

Wird beispielsweise nur eine Carbonsäure mit (3-Chlormethylphenyl)-chlorformiat in Kontakt gebracht, so reagiert nur die Chlorformiatgruppe mit der Carbonsäure, die Chlormethylgruppe bleibt weitgehend unverändert. Durch anschließenden Zusatz von Carbonsäuresalz kann dann auch die Chlormethylgruppe zur Reaktion gebracht werden. Die stöchiometrisch erforderlichen Mengen an Carbonsäure und Carbonsäuresalz betragen in diesem Fall je 1 mol pro Mol (3-Chlormethylphenyl)-chlorformiat. Da beim Einsatz von flüssigen Carbonsäuren diese vorteilhafterweise auch als Lösungsmittel für die Acidolysereaktion dienen können, setzt man vorzugsweise 1 bis 20 mol Carbonsäure, insbesondere 1 bis 10 mol Carbonsäure und vorzugsweise 1 bis 5 mol Carbonsäuresalz, insbesondere 1 bis 2 mol Carbonsäuresalz, jeweils pro Mol (3-Chlormethylphenyl)-chlorformiat ein.

Wird beispielsweise das Carbonsäuresalz direkt entweder in Gegenwart oder Abwesenheit einer Carbonsäure mit 3-(Chlormethylphenyl)-chlorformiat zusammengebracht, wird durch die Acidolyse der Chlorformiat- und der Chlormethylgruppe je 1 mol Carbonsäuresalz verbraucht, so dass in diesem Fall stöchiometrisch 2 mol Carbonsäuresalz zur vollständigen Acidolyse notwendig sind. Vorzugsweise setzt man in diesem Fall 2 bis 5 mol Carbonsäuresalz, insbesondere 2 bis 3 mol Carbonsäuresalz, bezogen auf 1 mol (3-Chlormethylphenyl)-chlorformiat ein.

Das Carbonsäuresalz kann entweder als solches eingesetzt oder in situ aus einer Carbonsäure und einer Base hergestellt werden. Als Base kommen beispielsweise Alkali- und Erdalkalihydroxide, Alkali- und Erdalkalicarbonate und Ammoniak in Frage. Beispiele für Basen sind NaOH, KOH, LiOH, Na₂CO₃, K₂CO₃, Mg(OH)₂, Ca(OH)₂ und/oder NH₃. Bevorzugt ist die Zugabe von Carbonsäuresalz als solchem.

Es können auch Gemische von verschiedenen Carbonsäuren und verschiedenen Carbonsäuresalzen eingesetzt werden. Bevorzugt ist jedoch die Anwendung einer Carbonsäure und eines ihrer Salze.

Die erfindungsgemäße Umsetzung von (3-Chlormethylphenyl)-chlorformiat mit der Carbonsäure und/oder dem Carbonsäuresalz kann gegebenenfalls in Gegenwart eines inerten Lösungsmittels durchgeführt werden. Als Beispiele seien genannt: aromatische und chloraromatische Kohlenwasserstoffe wie Benzol, Toluol, Xylol, Chlorbenzol, Chlortoluole, Dichlorbenzole und Trichlorbenzole, veretherte Aromaten wie Anisol, substituierte Anisole und Phenethol, aromatische Nitrile wie Benzonitril, Tolunitrile und Chlorbenzonitrile, hydrierte aromatische Kohlenwasserstoffe wie Decalin und/oder aliphatische Polyether wie 1,2-Dimethoxyethan.

Bevorzugt ist die Durchführung der Acidolyse in einem Überschuss an flüssiger Carbonsäure als Lösungsmittel.

Die erfindungsgemäße Acidolyse kann z.B. bei Temperaturen im Bereich von 20 bis 200°C, bevorzugt von 50 bis 150°C, durchgeführt werden.

Der Druck während der Acidolysereaktion kann erhöht, erniedrigt oder normal sein. Bevorzugt sind Drucke im Bereich 0,9 bis 3 bar.

Die in der ersten Reaktionsstufe hergestellten Bisester können aus dem Reaktionsgemisch z.B. durch Destillation oder wässrige Aufarbeitung isoliert werden. Gegebenenfalls können dabei anfallende anorganische Salze durch Wäsche mit Wasser entfernt und der Bisester durch Phasentrennung isoliert werden.

Erfindungsgemäß erhält man durch die in der ersten Stufe durchgeführten Acidolyse Bisester des 3-Hydroxybenzylalkohols. Diese Bisester enthalten in der Regel durch Teilhydrolyse bei der wässrigen Aufarbeitung und/oder durch Wasser, das bei der Acidolysereaktion anwesend ist, bereits einen gewissen Anteil an einem Monoester des 3-Hydroxybenzylalkohols, der aber bei der folgenden Alkoholyse in gleicher Weise wie der Bisester zum 3-Hydroxybenzylalkohol umgewandet wird. Der Gehalt an Monoester kann z.B. zu 50 Gew.-% betragen.

Erfindungsgemäß werden diese Bisester durch Umsetzung mit einem Alkohol (= Alkoholyse) in 3-Hydroxybenzylalkohol überführt.

Als Alkohole für diese Alkoholyse können z.B. aliphatische Alkohole mit 1 bis 12 C-Atomen oder cycloaliphatische Alkohole mit 5 bis 12 C-Atomen eingesetzt werden. Bevorzugt sind lineare und verzweigte aliphatische Alkohole mit 1 bis 6 C-Atomen und cycloaliphatische Alkohole mit 5 bis 8 C-Atomen. Beispielsweise seien genannt: Methanol, Ethanol, n-Propanol, i-Propanol, n-Butanol, i-Butanol, t-Butanol, n-Pentanol, n-Hexanol, Cyclohexanol und Methylcyclohexanole.

Als Alkohole können auch aromatische Alkohole mit beispielsweise 6 bis 10 C-Atomen eingesetzt werden wie Phenol, Kresole, Xylenole und Naphthole.

Die aliphatischen, cycloaliphatischen und aromatischen Alkohole können gegebenenfalls substituiert sein, beispielsweise mit Halogenatomen wie Fluor, Chlor oder Brom, bevorzugt Fluor oder Chlor. Die Substitution kann einfach oder mehrfach sein.

Die stöchiometrisch erforderliche Menge an einzusetzendem Alkohol beträgt 2 mol pro Mol Bisester des 3-Hydroxybenzylalkohols. Da sich jedoch in der Regel Gleichgewichte zwischen dem Bisester, den beiden monodeacylierten Zwischenstufen, dem 3-Hydroxybenzylalkohol, dem zugegebenen Alkohol und dem jeweiligen Carbonsäureester des Alkohols einstellen, ist es vorteihaft, für eine vollständige Umsetzung zum gewünschten 3-Hydroxybenzylalkohol die Gleichgewichte zum 3-Hydroxybenzylalkohol hin zu verschieben, was beispielsweise durch den Einsatz eines Überschusses an Alkohol oder durch Entfernung (z.B. durch Destillation) des gebildeten Alkohol-carbonsäureesters geschehen kann. Da im letzteren Falle häufig Azeotrope zwischen dem Alkohol und seinem Carbonsäureester vorliegen, ist es in beiden Fällen von Vorteil, den Alkohol im Überschuss einzusetzen, beispielsweise in Mengen von 2,1 bis 20 mol, bevorzugt 2,5 bis 10 mol pro Mol Bisester des 3-Hydroxybenzylalkohols.

Zur Beschleunigung der Alkoholyse des Bisesters kann man Katalysatoren zusetzen. Als Katalysatoren kann man z.B. solche einsetzen, die zur Alkoholyse von Carbonsäureestern bekannt sind, beispielsweise Basen, Säuren und Metallsalze oder Metallverbindungen. Als Basen kommen beispielsweise in Frage: Alkali- und Erdalkalihydroxide, Alkali- und Erdalkalicarbonate, Amine und Phosphine, insbesondere LiOH, NaOH, KOH, Na₂CO₃, K₂CO₃, Mg(OH)₂ und Ca(OH)₂. Als Säuren kommen beispielsweise in Frage: Mineral-, Carbon- und Sulfonsäuren, insbesondere H₂SO₄, H₃PO₄, HCI, HBr, HI, Essigsäure, Propionsäure und Benzoesäure. Als Metallsalze und Metallverbindungen kommen beispielsweise Salze und Verbindungen von Thallium, Zinn und Titan wie TlNO₃, (Butyl)₂SnO und Ti(O-Phenyl)₄.

Die Katalysatoren können auch in immobilisierter Form vorliegen, beispielsweise in Form basischer oder saurer Ionentauscher, die beispielsweise Amin- oder HSO₃-Funktionen oder das entsprechende Metall als Kation tragen.

Katalysatoren kann man, bezogen auf den Bisester des 3-Hydroxybenzylalkohols, beispielsweise in Mengen von 0,05 bis 10 mol-%, vorzugsweise 0,5 bis 3 mol-%, einsetzen. Wenn man innerhalb dieser Bereiche relativ geringe Mengen Katalysator einsetzt, vereinfacht sich die Aufarbeitung des Alkoholyse-Reaktionsgemisches.

Im Falle immobilisierter Katalysatoren bezieht sich der Begriff mol-% auf die aktiven funktionellen Gruppen des Katalysators.

Bevorzugt werden in die erfindungsgemäße Alkoholyse saure Katalysatoren wie H₂SO₄, H₃PO₄, HCl, HBr oder saure Ionentauscher eingesetzt.

Die Alkoholyse des Bisesters des 3-Hydroxybenzalkohols kann gegebenenfalls in Gegenwart inerter Lösungsmittel durchgeführt werden. Beispielsweise kommen die gleichen Lösungsmittel in Frage, die oben für die Acidolyse von (3-Chlormethylphenyl)-chlorformiat mit Carbonsäure und/oder deren Salzen angegeben wurden. Bevorzugt wird die Acidolyse jedoch ohne Lösungsmittelzusätze durchgeführt.

Als Alkohole können auch Mischungen verschiedener Alkohole und als Katalysatoren Mischungen verschiedener Katalysatoren eingesetzt werden.

Die Alkoholyse des Bisesters des 3-Hydroxybenzylalkohols kann z.B. bei Temperaturen im Bereich 0 bis 200°C, bevorzugt von 50 bis 150°C, durchgeführt werden.

Der Druck bei der Alkoholysereaktion kann erhöht, erniedrigt oder normal sein. Bevorzugt sind Normaldruck, erniedrigte Drucke, die gegebenenfalls zum Abdestillieren eines höhersiedenden Carbonsäureesters erforderlich sind und Überdrucke, die gegebenenfalls erforderlich sind zur Überwindung eines Azeotropes. Dementsprechend liegt der Reaktionsdruck z.B. bei 0,01 bis 10 bar, bevorzugt bei 0,1 bis 5 bar.

Das erfindungsgemäße Verfahren kann in verschiedenen Ausführungsformen durchgeführt werden. Grundsätzlich kann jeder der beiden Teilschritte kontinuierlich, diskontinuierlich oder teilkontinuierlich in Schüben durchgeführt werden.

In einer bevorzugten Ausführungsform wird die Carbonsäure (z.B. Essigsäure) vorgelegt, auf Rückflußtemperatur erhitzt und unter Rühren (3-Chlormethylphenyl)-chlorformiat eindosiert, wobei im Wesentlichen nur die Chlorformiatgruppe acidolysiert wird. In das ausreagierte Gemisch wird dann das Carbonsäuresalz (z.B. Natriumacetat) gegeben und in der Hitze nachgerührt. Dabei wird die Chlormethylgruppe acidolysiert. Man erhält so nach beispielsweise wässriger Aufarbeitung den Bisessigsäureester des 3-Hydroxybenzylalkohols.

Bei der wässrigen Aufarbeitung wird z.B. vom Reaktionsgemisch die als Lösungsmittel vorhandene Carbonsäure abdestilliert und der dabei anfallende Rückstand zur Entfernung der Salze mit Wasser verrührt.

Durch Hydrolyse während dieser Aufarbeitung, aber auch durch Hydrolyse durch Wasser, das bei der Acidolysereaktion vorhanden sein kann, kann der Bisester zum Teil schon zu einem Monoester des Hydroxybenzylalkohols deacyliert werden. Dieses Monoacylierungsprodukt des 3-Hydroxybenzylalkohols fällt im Gemisch mit dem Bisester an. Es ist eines der Zwischenprodukte bei der nachfolgenden Alkoholyse des Bisesters zum 3-Hydroxybenzylalkohol, so dass es ebenfalls zum gewünschten Produkt 3-Hydroxybenzylalkohol umgewandelt wird.

Zum hergestellten Bisester gibt man dann Katalysator und einen Überschuss an Alkohol (z.B. Methanol oder Ethanol) zu und destilliert den gebildeten Carbonsäureester (z.B. Essigsäuremethyl- oder -ethylester) als Azeotrop mit dem Alkohol ab. Gegebenenfalls kann zur vollständigen Umsetzung Alkohol während der Destillation in das Reaktionsgemisch kontinuierlich oder teilkontinuierlich in Schüben nachdosiert werden. Nach der Umsetzung wird dann der Alkohol zusammen mit restlichen Carbonsäureester abgedampft. Der zurückbleibende Rückstand kann erforderlichenfalls durch Umkristallisation gereinigt werden.

In einer anderen bevorzugten Ausführungsform wird ein Gemisch aus Carbonsäure (z.B. Essigsäure) und ihrem Salz (z.B. Natriumacetat) vorgelegt und in der Hitze (3-Chlormethylphenyl)-chlorformiat eindosiert. Nach vollständiger Umsetzung und wässriger Aufarbeitung erhält man auch so den Bisessigsäureester des 3-Hydroxybenzylalkohols der dann wie oben beschrieben alkoholisiert werden kann.

Das erfindungsgemäße Verfahren hat durch den Übergang auf ein für diesen Zweck völlig neuartiges Ausgangsmaterial eine Reihe von Vorteilen. Zum Einen wird einfach und kostengünstig zur Verfügung stehendes (3-Chlormethylphenyl)-chlorformiat als Ausgangsmaterial eingesetzt. Zum Anderen werden einfache Hilfsmittel benötigt wie Carbonsäuren, Carbonsäuresalze und Alkohole, die kostengünstig zugänglich sind und keinen besonderen Aufwand in ihrer Handhabung erfordern. Mit dem erfindungsgemäßen Verfahren kann deshalb 3-Hydroxybenzylalkohol auf wesentlich einfachere und kostengünstigere Weise erhalten werden als bisher.

### Beispiel

In einem Reaktionsgefäß wurden 100 Gew.-Teile Essigsäure vorgelegt und bei 120°C gerührt. Dann wurden 60,3 Gew.-Teile (3-Chlormethylphenyl)-chlorformiat (Gehalt gemäß GC-Area in %: 98,0) im Verlauf von einer Stunde zudosiert. Nunmehr wurde 1 Stunde bei 120 bis 125°C nachgerührt, danach die Mischung auf 80°C abgekühlt. Jetzt wurden unter Rühren 31,9 Gew.-Teile Natriumacetat zugefügt und 7 weitere Stunden am Rückfluss gerührt. Nachdem der größte Teil der noch vorhandenen Essigsäure zunächst beim Normaldruck, zuletzt im Vakuum bis 23 mbar abdestilliert worden war, wurde der Rückstand in 100 Gew.-Teilen Wasser aufgenommen, die organische Phase abgetrennt und nochmals mit 22 Gew.-Teilen Wasser gewaschen. Die wässrigen Phasen wurden verworfen. Man erhielt so 58,2 Gew.-Teile eines öligen Produktes, das gemäß GC-Analyse zu 78,3 % aus dem Bisessigsäureester des 3-Hydroxybenzylalkohols und zu 19,2 % aus einem monoacetylierten Derivat des 3-Hydroxybenzylalkohols bestand.

Dieses Gemisch wurde mit 55,4 Gew.-Teilen Methanol und 0,58 Gew.-Teilen konzentrierter Schwefelsäure versetzt und in einer Kolonne (10 theoretische Böden) zum Rückfluss erhitzt. Im Verlaufe von 4 Stunden wurden 75 Gew.-Teile Methanol/Methylacetat/Azeotrop abgenommen und die gleiche Menge Methanol in den Reaktionssumpf nachdosiert.

Man erhielt so 104,8 Gew.-Teile einer methanolischen Lösung von 3-Hydroxybenzylalkohol mit einem Gehalt von 32,2 % 3-Hydroxybenzylalkohol (HPLC). Die Ausbeute betrug somit 94,4 % der Theorie bezogen auf eingesetztes (3-Chlormethylphenyl)-chlorformiats.

Der hergestellte 3-Hydroxybenzylalkohol kann durch Abdampfen des Methanols und Umkristallisation, beispielsweise aus Benzol oder Tetrachlorkohlestoff, in reiner Form isoliert werden.

## Patentansprüche

1. Verfahren zur Herstellung von 3-Hydroxybenzylalkohol, **dadurch gekennzeichnet, dass** man (3-Chlormethylphenyl)-chlorformiat mit einer Carbonsäure und/oder einem Carbonsäuresalz einer Acidolyse unterwirft und anschließend mit dem Reaktionsprodukt dieser Stufe eine Alkoholyse durchführt.

2. Verfahren nach Anspruch 1, **dadurch gekennzeichnet, dass** man (3-Chlormethylphenyl)-chlorformiat einsetzt, das weniger als 1 Gew.-% nicht seitenkettenchloriertes (3-Methylphenyl)-chlorformiat und weniger als 5 Gew.-% höherchloriertes (3-Bis-chlormethylphenyl)-chlorformiat enthält.

3. Verfahren nach Ansprüchen 1 und 2, **dadurch gekennzeichnet, dass** man die Acidolyse mit einer Carbonsäure durchführt, die lineare oder verzweigte Alkylreste mit 1 bis 12 C-Atomen enthält oder mit einer cycloaliphatischen Carbonsäure, die 4 bis 11 C-Atome enthält, oder mit einer aromatischen Carbonsäure, die 7 bis 12 C-Atome enthält.

4. Verfahren nach Ansprüchen 1 bis 3, **dadurch gekennzeichnet, dass** man eine Carbonsäure mit (3-Chlormethylphenyl)-chlorformiat in Kontakt bringt und anschließend Carbonsäuresalz zusetzt und dabei 1 bis 20 mol Carbonsäure und 1 bis 5 mol Carbonsäuresalz einsetzt.

5. Verfahren nach Ansprüchen 1 bis 3, **dadurch gekennzeichnet, dass** man Carbonsäuresalz direkt mit (3-Chlormethylphenyl)-chlorformiat in Kontakt bringt und dabei 2 bis 5 mol Carbonsäuresalz einsetzt.

6. Verfahren nach Ansprüchen 1 bis 5, **dadurch gekennzeichnet, dass** man die Acidolyse bei Temperaturen im Bereich 20 bis 200°C und Drucken im Bereich 0,9 bis 3 bar durchführt.

7. Verfahren nach Ansprüchen 1 bis 6, **dadurch gekennzeichnet, dass** man die Alkoholyse mit einem aliphatischen Alkohol mit 1 bis 18 C-Atomen, einem cycloaliphatischen Alkohol mit 5 bis 12 C-Atomen oder einem aromatischen Alkohol mit 6 bis 11 C-Atomen durchführt.

8. Verfahren nach Ansprüchen 1 bis 7, **dadurch gekennzeichnet, dass** man die Alkoholyse mit 2,1 bis 20 mol eines Alkohols, bezogen auf 1 mol des als Zwischenprodukt gebildeten Bisesters des 3-Hydroxybenzylalkohols durchführt.

9. Verfahren nach Ansprüchen 1 bis 8, **dadurch gekennzeichnet, dass** man die Alkoholyse in Gegenwart eines Katalysators durchführt.

10. Verfahren nach Ansprüchen 1 bis 9, **dadurch gekennzeichnet, dass** man die Alkoholyse bei Temperaturen im Bereich 0 bis 200°C und bei Drucken im Bereich von 0,01 bis 10 bar durchführt.
